# EUROPEAN PATENT SPECIFICATION

(11) **EP 0 868 507 B1**
(45) Date of publication and mention of the grant of the patent: **31.03.2004**
(21) Application number: 96943266.5
(22) Date of filing: 20.12.1996
(51) Int. Cl.: C12N 9/80, C07K 1/16

(54) **PROCESS FOR THE PURIFICATION OF UREASE FROM HELICOBACTER PYLORI**
VERFAHREN ZUR REINIGUNG DER UREASE AUS HELICOBACTER PYLORI
PROCEDE DE PURIFICATION DE L'UREASE A PARTIR DE HELICOBACTER PYLORI

(30) Priority: 20.12.1995 DE 19547708
(43) Date of publication of application: 07.10.1998
(73) Proprietor: Chiron Behring GmbH & Co., 35006 Marburg (DE)
(72) Inventor: LENZ, Uwe, D-35083 Wetter (DE)
(74) Representative: Hallybone, Huw George
(86) International application number: PCT/IB1996/001483
(87) International publication number: WO 1997/022693

(56) References cited:
- US-A- 4 336 161
- INFECT IMMUN, DEC 1992, 60 (12) P5259-66, UNITED STATES, XP000647859 TURBETT GR ET AL: "Purification and characterization of the urease enzymes of Helicobacter species from humans and animals." cited in the application
- MICROB PATHOG, JAN 1991, 10 (1) P15-26, ENGLAND, XP000646791 EVANS DJ JR ET AL: "Characterization of the Helicobacter pylori urease and purification of its subunits."
- J BIOL CHEM, JUN 5 1990, 265 (16) P9464-9, UNITED STATES, XP000568708 DUNN BE ET AL: "Purification and characterization of urease from Helicobacter pylori."
- INFECTION AND IMMUNITY, vol. 58, no. 4, 1990, WASHINGTON US, pages 992-998, XP002029368 HU L-T ET AL: "Purification and N-terminal analysis of urease from Helicobacter pylori" cited in the application

## Description

The invention relates to a novel process for the purification of urease from Helicobacter pylori, in which the urease is purified from a cell-free extract by chromatography on a dye ligand chromatography material.

Helicobacter pylori is a helical human pathogenic rod which populates the mucus layer of the antrum region of the stomach. Investigations in recent years have shown that an infection with Helicobacter pylori in man may be associated with an increased prevalence of gastric and duodenal ulcers. Furthermore, infection with this microorganism is a principal risk factor for the development of gastric carcinomas.
Characteristic of bacteria of the species Helicobacter pylori is the production of large amounts of a urease (EC 3.5.1.5) with high activity. It is suspected that this enzyme makes possible the population of the stomach by the bacteria by protecting the bacteria from the gastric acid by the production of ammonia. The urease is a symmetrical hexamer having a molecular weight of about 550 kD, which is composed of two different subunits, UreA and UreB, with a molecular weight of about 30 kD and 64 kD respectively. In contrast to other bacterial ureases, which are cytoplasmic, the urease of Helicobacter pylori appears to be associated with the cell surface (Hawtin et al., J. Gen. Microbial. 136 (1990), 1995-2000). Experiments in the mouse model showed that the urease is a protective antigen and thus suitable as a constituent of a vaccine (Michetti et al., Gastroenterology 107 (1994), 1002-1011). A requirement for the use of the urease in a vaccine is the preparation of large amounts of this protein in pure form. This necessitates processes which allow an inexpensive and efficient purification of the enzyme. Different processes for the purification of the urease from Helicobacter pylori have already been described which, however, are not suitable for the preparation of large amounts of the enzyme. The purification described by Hu and Mobley (Infect. Immun. 58 (1990), 992-998) comprises, for example, a chromatographic purification in four stages (anion-exchange chromatography, HIC chromatography, cation-exchange chromatography and gel filtration) and is thus very cost- and time-intensive. The purification of the urease by means of affinity chromatography on columns which are coupled to monoclonal antibodies against urease (see, for example, Turbett et al., FEMS Microbiol. Immunol. 76 (1991), 19-24; Turbett et al., Infect. Immun. 60 (1992), 5259-5266), necessitates, on the one hand, the production of large amounts of monoclonal antibodies and, on the other hand, has the disadvantage that the purified product is contaminated to a certain extent with monoclonal antibodies.

The present invention is thus based on the object of making available a simple and efficient process for the purification of urease from Helicobacter pylori.

This object is achieved by the preparation of the embodiments described in the patent claims.

The invention thus relates to a process for the purification of urease from Helicobacter pylori, in which
(i) a urease-containing extract from Helicobacter pylori is brought into contact with a dye ligand chromatography material in a magnesium-containing buffer with a pH in the acidic range; and
(ii) the urease bound to the dye ligand chromatography material is eluted.

The process according to the invention is based on the surprising effect that the urease from Helicobacter pylori remains in solution when the pH is lowered if a defined amount of magnesium ions is present in the buffer used, and under the same conditions binds with high affinity to a dye ligand chromatography material. Under these conditions, the majority of the other proteins from Helicobacter pylori extracts precipitate from the solution and can thus be separated even before dye ligand chromatography. Furthermore, the urease bound to the dye ligand chromatography material can be eluted using a pH gradient, only a low contamination with foreign protein occurring. This makes it possible to obtain the urease in almost pure form even in a one-stage process and to prepare it in pure form by means of a subsequent purification step, for example by means of conventional anion-exchange chromatography.

The term urease-containing extract in the context of the present invention comprises any type of extracts of Helicobacter pylori which contain proteins with the biological activity of a urease. In this case, they are preferably cell-free extracts which can be obtained by disruption of the cells and separation of insoluble constituents. The preparation of a urease-containing extract of this type can be carried out, for example, by the processes described in the literature (see, for example, Turbett et al., FEMS Microbiol. Immunol. 76 (1991), 19-24; Turbett et al., Infect. Immun. 60 (1992), 5259-5266; Hu and Mobley, Infect. Immun. 58 (1990), 992-998). In this context, Helicobacter pylori cells are first cultured and harvested according to known processes. The cells resuspended in a suitable buffer are then disrupted in a hypotonic buffer by means of ultrasound or French press and cell debris is centrifuged off.

In a preferred embodiment of the process according to the invention, the urease-containing extract is obtained by disrupting the cells of a bacterial suspension of Helicobacter pylori by ultrasonic treatment and centrifuging off insoluble constituents. The buffer used in this case preferably has a pH in the neutral range and is magnesium-free. The concentration of magnesium ions necessary for the process according to the invention is preferably set by addition of magnesium ions after the ultrasonic treatment. This leads to an increase in the urease yield.

In the context of the present invention, the term dye ligand chromatography comprises any type of purification of the urease from Helicobacter pylori on a support material on which a dye is immobilized.

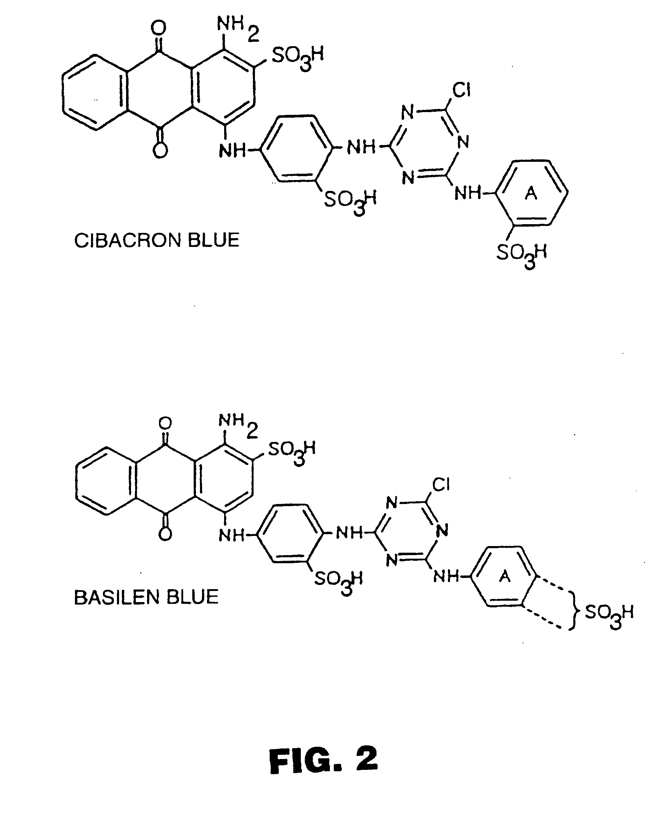

In a preferred embodiment, the dye coupled to the support material is a triazine dye. Dyes of this type contain ionized groups and a conjugated ring system and bind in a non-specific manner to the effector centres of proteins. Particularly preferred is the use of support materials to which the triazine dye Cibacron blue® (Ciba-Geigy) is coupled, as well as of support materials to which a Basilen dye (BASF) is coupled. Both Cibacron and Basilen dyes are reactive dyes based on monochlorotriazine and differ only by the anilinesulphonic acid substituted on the triazine ring, which occurs in three positional isomer forms. The structural formulae of the preferably used triazine dyes Cibacron blue® or Basilen blue® are shown in Figure 2. Possible support materials are in principle all support materials customarily used in chromatographic processes. An example of suitable dye ligand chromatography materials is the gel marketed under the trade name BLUEHyperD® (Bio Sepra), which contains as the ligand a Basilen dye (BASF) as well as the gel marketed as Blue Sepharose 6FF® (Pharmacia LKB), which contains as the ligand the dye Cibacron blue ®. In the case of BLUEHyperD®, a gel embedded in a polystyrene structure serves as a support, in the case of Blue Sepharose 6FF®, on the other hand, a pure agarose gel.

Preferably, the magnesium ion concentration of the urease-containing extract from Helicobacter pylori which is used for the purification of the urease is in a range from 15 mM to 25 mM, preferably 20 mM. The necessary magnesium ion concentration is preferably set after preparation of the urease-containing extract and before addition to the dye ligand chromatography material.

In a preferred embodiment of the process according to the invention, the pH of the urease-containing extract which is used for the purification of the urease is in a range from pH 3.8 to pH 7.8, preferably in a range from pH 4.8 to pH 5.0. In principle, to adjust the pH any organic or inorganic acid can be used. Acetic acid is preferably used for this purpose. The adjustment of the pH is preferably carried out after the required concentration of magnesium ions has been set.

At the magnesium ion concentrations and pH values indicated, it is guaranteed that the urease remains in solution, whereas a majority of the other proteins present in the extract precipitate. These are preferably separated off before the urease-containing extract is brought into contact with the dye ligand chromatography material, for example by centrifugation. This denotes a first purification step. Furthermore, it is guaranteed that the urease binds efficiently to the dye ligand chromatography material and remains bound during the washing of the material to remove non-bound proteins.

The purification of the urease from Helicobacter pylori with the aid of a dye ligand chromatography material can be carried out either in a batch process or using a column.
In the batch process, the urease-containing extract, for example, is directly incubated with the dye ligand chromatography material after separation of the precipitated proteins. A buffer is used here which has a magnesium ion concentration of preferably 3 to 30 mM, particularly preferably of 10 to 20 mM, and a pH in the ranges indicated above. After washing the dye ligand chromatography material several times with the buffer to remove unbound proteins, the urease bound to the material is eluted using a suitable buffer.
In the case of purification by column chromatography, the dye ligand chromatography material is packed, for example, in a suitable column and equilibrated with a buffer which has a magnesium ion concentration and a pH in the ranges indicated above. After application of the urease-containing extract, the column is thoroughly washed with the buffer. The urease bound to the column material is then eluted using a suitable buffer.

The elution of the urease can be carried out by increasing the salt concentration or the pH, e.g. by means of a stepwise or continuous gradient. On account of the more strongly occurring elution of foreign proteins likewise bound to the column, however, the elution of the urease by increasing the salt concentration may lead to a lower degree of purification.

In a preferred embodiment, therefore, the elution of the urease bound to the dye ligand chromatography material is carried out by increasing the pH. The pH of the buffer used for elution is in this case preferably above a pH of 5.0, particularly preferably in a range from pH 7.8 to pH 9.0. The use of a buffer having a pH of 7.8 or higher guarantees that the bound urease is eluted efficiently from the dye ligand chromatography material. The buffer used for elution can contain magnesium ions, preferably in a range from 3 to 30 mM, particularly preferably from 10 to 20 mM, or be free of magnesium ions.

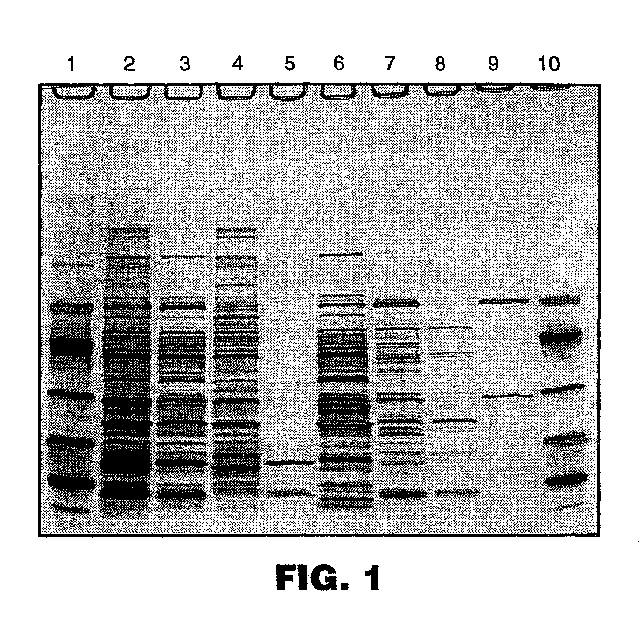

The purification of the urease from Helicobacter pylori by dye ligand chromatography already yields a largely pure urease with a low contamination by foreign protein (see Figure 1).

In a further preferred embodiment of the process according to the invention, a fine purification of the urease by a further chromatography step follows the purification by dye ligand chromatography. All chromatography processes customary in the field of protein purification are suitable in this context, inter alia also cation-exchange chromatography or HIC chromatography, gel filtration, etc.

In a particularly preferred embodiment, the fine purification of the urease is carried out by anion-exchange chromatography. To this end, the urease-containing eluate obtained in the dye ligand chromatography is applied directly or after concentration to an anion exchanger and, after the removal of unbound proteins, eluted by increasing the salt concentration. For elution, for example, buffers having an increased sodium ion concentration can be used.

Preferably, buffers having an increased magnesium ion concentration are used. This makes possible the selective elution of the urease by a single-stage gradient. In contrast to this, elution by means of increased sodium ion concentrations necessitates a continuously rising gradient. The magnesium ion concentration in the buffer used for elution is preferably in the range from 30 to 50 mM and particularly preferably 40 mM. The use of buffers of increased magnesium ion concentration for elution of the urease from an anion exchanger makes possible both a simplification of the purification process and a higher degree of purity of the eluted urease.
With the aid of purification by anion exchange chromatography, contaminations by foreign proteins are efficiently removed and the urease is obtained in pure form (see Figure 1).
The material of the anion exchanger used can in principle be any anion exchanger customarily used in chromatography processes. Preferably, anion exchangers are used which have a quaternary ammonium group. An example of an anion exchanger of this type which is particularly preferred in the context of the present invention is the material marketed by Pharmacia LKB under the name RESOURCE Q®.

In comparison with the previously known processes for the purification of urease from Helicobacter pylori or from other bacteria, the described process by means of dye ligand chromatography offers the advantage of a simple and rapid isolation of the urease in high yield. Furthermore, the described process is inexpensive, as the material for dye ligand chromatography is a frequently reusable material.

### Description of the figures

Figure 1 shows an SDS gel electrophoresis of samples of various purification steps of the purification of the urease from Helicobacter pylori described in Examples 1, 2 and 3.
- Track 1:: 5 µl of low marker (Pharmacia)
- Track 2:: 5 µl of crude extract
- Track 3:: 5 µl of supernatant after centrifugation
- Track 4:: 5 µl of residue after centrifugation
- Track 5:: 5 µl of runnings of BLUE HyperD
- Track 6:: 5 µl of eluate of the regeneration with 1 M NaCl of BLUE HyperD
- Track 7:: 5 µl of urease eluate of BLUE HyperD
- Track 8:: 5 µl of runnings of RESOURCE Q (in 100 mM NaCl)
- Track 9:: 5 µl of urease eluate of RESOURCE Q
- Track 10:: 5 µl of low marker, 1:20 (Pharmacia)

| Marker: | |
|---|---|
| Phosphorylase b | 94 kD |
| Albumin | 67 kD |
| Ovalbumin | 43 kD |
| Carbonic anhydrase | 30 kD |
| Trypsin inhibitor | 20.1 kD |
| α-Lactalbumin | 14.4 kD |

Figure 2 shows the structural formulae of the triazine dyes Cibacron blue® and Basilen blue®.

The examples illustrate the invention.

### Example 1

### Preparation of a cell-free urease-containing extract of Helicobacter pylori

If not stated otherwise, all steps in the purification of the urease were carried out at room temperature. To prepare a cell-free extract, bacteria of the species Helicobacter pylori were cultured according to known methods. 200 ml of a bacteria-containing suspersion were centrifuged off and washed several times with 20 mM tris/HCl buffer (pH 7.0). The cell precipitate was taken up in 20 ml of 20 mM tris/BCl buffer (pH 6.9). The bacterial cells were disrupted by ultrasonic treatment and the insoluble constituents were removed as a sediment by centrifugation. The urease-containing supernatant (crude extract) was used as a starting material for the purification of the urease by chromatographic processes.

### Example 2

### Purification of the urease from Helicobacter pylori by dye ligand chromatography

To purify the urease, the magnesium ion concentration of the crude extract obtained according to Example 1 was first adjusted to 20 mM using a 1 M MgCl₂ solution. The pH was then adjusted to pH 4.8. To do this, dilute acetic acid (0.25%) was added with stirring. In this process, a majority of the foreign proteins precipitated. These were separated off by centrifugation (10 min at 3000 rpm; 4°C). Under these conditions, the urease remained in solution and was found in the supernatant. The supernatant was used for purification by dye ligand chromatography.

The material BLUEHyperD® from Bio Sepra was used for dye ligand chromatography and was packed into a column (26 mm × 150 mm; gel height 16 mm). Before the application of the urease-containing extract, the column was equilibrated with 10 mM acetate buffer, 10 mM MgCl₂·6H₂O, pH 4.8. Column chromatography was carried out with the aid of an FPLC apparatus (Pharmacia LKB). The elution of proteins was detected by means of a UV detector.

The urease-containing supernatant was applied to the equilibrated column. After application, the column was washed with the equilibration buffer until proteins were no longer detectable in the runnings. The elution of the urease bound to the dye ligand chromatography column was carried out with the aid of 20 mM tris/HCl buffer, pH 7.8. Protein-containing fractions were collected.
For documentation of the purification, samples were removed in the individual purification steps, treated under denaturing conditions and applied to a 4-20% strength tris/glycine gradient gel (according to Laemmli). Detection was carried out by means of silver staining by methods known to the person skilled in the art.
As can be seen from Figure 1, dye ligand chromatography already yields a relatively pure urease. The two readily visible bands in the range of 67 and 30 kD are the subunits UreB and UreA of the urease eluted from the dye ligand chromatography column.

### Example 3

### Fine purification of the urease by anion-exchange chromatography

In order to purify the substantially pure urease obtained by dye ligand chromatography from residual foreign proteins, the eluate of the BLUEHyperD® column was purified further by anion-exchange chromatography. The chromatography was again carried out with the aid of an FPLC unit (Pharmacia LKB). The urease-containing eluate of the dye ligand chromatography column was applied directly to a column (6.4 mm × 30 mm; gel bed volume 1 ml) equilibrated with 20 mM tris/HCl, pH 7.2, 100 mM NaCl, which as an anion exchanger contained the material RESOURCE Q® (Pharmacia LKB), at a flow rate of 190 cm/h. The column was then washed with the equilibration buffer until proteins were no longer detectable in the runnings. The elution of the urease bound to the column was carried out with the aid of 20 mM tris/HCl buffer, 40 mM MgCl₂, pH 7.2. The protein-containing fractions were collected and analysed by means of SDS polyacrylamide gel electrophoresis.

As can be seen from Figure 1, the urease purified further by anion exchange chromatography is highly pure, and in an SDS gel, besides the two subunits of the urease, nearly no foreign proteins can be detected after silver staining.

The efficiency of the process according to the invention is clear from the following tables. A purification table for the purification steps described in Examples 1 to 3 is shown.

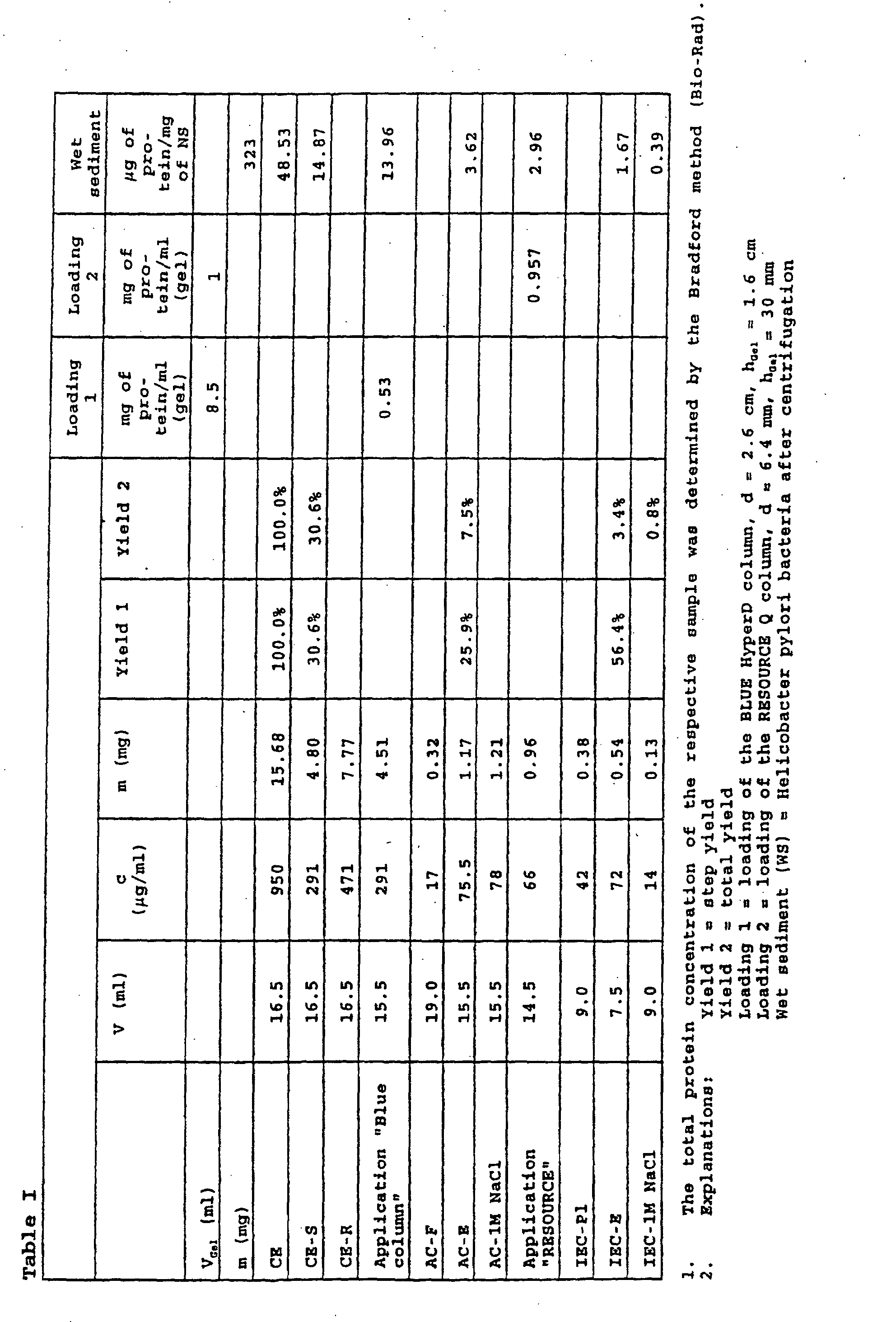

The yield of urease was determined by densitometric measurement.

The following table shows the urease yields of a particular chromatographic step determined in the quantitative assessment of an SDS gel (22% strength tris-glycine gel according to Laemmli; staining: Coomassie Blue). The measurements were carried out using a dual-wavelength TLC scanner CS-930 (Shimadzu).

**Table II**

| 64 kD urease band | | | | | |
|---|---|---|---|---|---|
| | Fraction | V (ml) | Area measured | Area total | Field (urease) |
| BLUE HyperD | CE-S (application) | 15.5 | 11337 | 175723.5 | |
| | AC-E | 15.5 | 8487 | 131548.5 | 74.86% |
| | AC-1M NaCl | 15.5 | 1650 | 25575 | |
| RESOURCE Q | AC-E (application) | 14.5 | 7487 | 108561.5 | |
| | IEC-P1 | 9 | 1264 | 11376 | |
| | IEC-E | 7.5 | 9604 | 72030 | 66.35% |
| | IEC-1M NaCl | 9 | 745 | 6705 | |
| Abbreviations used in the Tables: CE (crude extract) = raw material S = supernatant R = residue Blue column = Blue HyperD column AC = affinity chromatography F = runnings E = eluate 1 M NaCl = eluate with 1 M NaCl solution IEC = ion-exchange chromatography P1 = peak/after washing with equilibration buffer (100 mM NaCl) | | | | | |

## Claims

1. Process for the purification of urease from Helicobacter pylori, **characterized in that**
(i) a urease-containing extract from Helicobacter pylori is brought into contact with a dye ligand chromatography material in a magnesium-containing buffer with a pH in the acidic range; and
(ii) the urease bound to the dye ligand chromatography material is eluted.

2. Process according to Claim 1, the urease-containing extract being obtained by ultrasonic treatment of a bacterial suspension of Helicobacter pylori and subsequent centrifugation.

3. Process according to Claim 1 or 2, the dye ligand chromatography material being a matrix coupled to a triazine dye.

4. Process according to Claim 3, the triazine dye being the dye Cibacron blue® or Bastion blue®.

5. Process according to one of Claims 1 to 4, the concentration of magnesium ions in the buffer used being between 15 and 25 mM.

6. Process according to one of Claims 1 to 5, the pH of the buffer used being in the range from pH 3.8 to pH 7.8.

7. Process according to Claim 6, the pH being in the range from 4.8 to 5.0.

8. Process according to one of Claims 1 to 7, the elution of the urease from the dye ligand chromatography material being carried out by increasing the pH.

9. Process according to Claim 8, the buffer used for elution containing magnesium ions.

10. Process according to one of Claims 1 to 9, the urease purified by dye ligand chromatography being further purified by subsequent anion-exchange chromatography.

11. Process according to Claim 10, the anion-exchange chromatography material having quaternary ammonium groups.

12. Process according to Claim 11, the anion-exchange chromatography material being the material RESOURCE Q .

## Patentansprüche

1. Verfahren zur Reinigung von Urease aus Helicobacter pylori, **dadurch gekennzeichnet, dass**
(i) ein Urease-enthaltender Extrakt von Helicobacter pylori mit einem Farbstoffligandenchromatographiematerial in einem Magnesium-enthaltenden Puffer mit einem pH-Wert im saueren Bereich in Kontakt gebracht wird; und
(ii) die an das Farbstoffligandenchromatographiematerial gebundene Urease eluiert wird.

2. Verfahren gemäß Anspruch 1, wobei der Urease-enthaltende Extrakt durch Ultraschallbehandlung einer Bakteriensuspension von Helicobacter pylori und anschließendes Zentrifugieren erhalten wird.

3. Verfahren gemäß Anspruch 1 oder 2, wobei das Farbstoffligandenchromatographiematerial eine Matrix ist, die an einen Triazinfarbstoff gekoppelt ist.

4. Verfahren gemäß Anspruch 3, wobei der Triazinfarbstoff der Farbstoff Cibacron-Blau® or Basilen-Blau® ist.

5. Verfahren gemäß einem der Ansprüche 1 bis 4, wobei die Konzentration an Magnesiumionen in dem verwendeten Puffer zwischen 15 und 25 mM liegt.

6. Verfahren gemäß einem der Ansprüche 1 bis 5, wobei der pH-Wert des verwendeten Puffers im Bereich von pH 3,8 bis pH 7,8 liegt.

7. Verfahren gemäß Anspruch 6, wobei der pH-Wert im Bereich von 4,8 bis 5,0 liegt.

8. Verfahren gemäß einem der Ansprüche 1 bis 7, wobei die Elution der Urease von dem Farbstoffligandenchromatographiematerial durch Erhöhen des pH-Werts erfolgt.

9. Verfahren gemäß Anspruch 1 bis 8, wobei der zur Elution verwendetet Puffer Magnesiumionen enthält.

10. Verfahren gemäß einem der Ansprüche 1 bis 9, wobei die durch Farbstoffligandenchromatographie gereinigte Urease ferner durch anschließende Anionenaustauschchromatographie gereinigt wird.

11. Verfahren gemäß Anspruch 10, wobei das Anionenaustauschchromatographiematerial quaternäre Ammoniumgruppen aufweist.

12. Verfahren gemäß Anspruch 11, wobei das Anionenaustauschchromatographiematerial das Material RESOURCE Q ist.

## Revendications

1. Procédé de purification de l'uréase à partir d'*Helicobacter pylori*, **caractérisé en ce que**
(i) un extrait d'*Helicobacter pylori* contenant de l'uréase est mis en contact avec un matériel de chromatographie à ligand colorant dans un tampon contenant du magnésium ayant un pH compris dans le domaine acide ; et
(ii) l'uréase liée au matériel de chromatographie à ligand colorant ie est éluée.

2. Procédé selon la revendication 1, l'extrait contenant de l'uréase étant obtenu par traitement aux ultrasons d'une suspension bactérienne d'*Helicobacter pylori* puis centrifugation.

3. Procédé selon la revendication 1 ou la revendication 2, le matériel de chromatographie à ligand colorant étant une matrice couplée à un colorant triazine.

4. Procédé selon la revendication 3, le colorant triazine étant le colorant Cibacron blue® ou Basilen blue®.

5. Procédé selon l'une quelconque des revendications 1 à 4, la concentration en ions magnésium dans le tampon utilisé étant comprise entre 15 et 25 mM.

6. Procédé selon l'une quelconque des revendications 1 à 5, le pH du tampon utilisé étant compris entre pH 3,8 et pH 7,8.

7. Procédé selon la revendication 6, le pH étant compris entre 4,8 et 5,0.

8. Procédé selon l'une quelconque des revendications 1 à 7, l'élution de l'uréase à partir du matériel de chromatographie à ligand colorant étant réalisée par une augmentation du pH.

9. Procédé selon la revendication 8, le tampon utilisé pour l'élution contenant des ions magnésium.

10. Procédé selon l'une quelconque des revendications 1 à 9, l'uréase purifiée par chromatographie à ligand colorant étant purifiée une nouvelle fois par chromatographie ultérieure par échange d'anions.

11. Procédé selon la revendication 10, le matériel de chromatographie par échange d'anions contenant des groupes ammonium quaternaire.

12. Procédé selon la revendication 11, le matériel de chromatographie par échange d'anions étant le matériel RESOURCE Q.
